# EUROPEAN PATENT APPLICATION

(11) **EP 3 381 419 A1**
(43) Date of publication of application: **03.10.2018**
(21) Application number: 17163962.8
(22) Date of filing: 30.03.2017
(51) Int. Cl.: A61F 7/03, A61F 7/00, A61F 7/02

(54) **DEVICE FOR HEAT THERAPY OF SKIN DISORDERS**

(71) Applicant: Trimb Healthcare AB, 114 26 Stockholm (SE)
(72) Inventor: VAN EIBERGEN SANTHAGENS, Ivo, 1059 AT Amsterdam (NL); STAL, Robert Sebastian, 1018 HA Amsterdam (NL); WITTE, Johannes Hendricus, 2111 BN Aerdenhout (NL); KNEPPERS, Job Leonardus, 2613 PZ Delft (NL)
(74) Representative: Brann AB

(57) **Abstract**

A device (1, 60) for the treatment of skin disorders the device comprising a layer of a thermally insulating material (14, 74) with an opening (18, 78), and a heat conductor (15, 66) at one side of the opening. The device can for example comprise a plaster with a heat conductive top layer and a docking station comprising a heater to be contacted with the top layer of the plaster. Alternatively, the device may comprise a heat pack.

## Description

The present disclosure relates to a device for thermal treatment of skin, for example for the treatment of skin disorders, such as warts.

WO 01/58408 discloses heat therapy systems for the treatment such as heat delivery patches and exothermic pads. Other examples of heat therapy pad or plasters are disclosed in WO 2006/002949, US 5,053,024 and US 2004/0096488.

A drawback of existing heat delivery patches is that the applied heat is distributed over a relatively large area of the patient's skin. Surrounding tissue may not be harmed so the applied temperature cannot be too high. The effectiveness of exiting heat treatment pads is therefore limited.

It is an object of the present invention to provide a heat treatment device with improved effectiveness.

The object of the invention is achieved with a device for the treatment of skin disorders the device comprising:
- a layer of a thermally insulating material (14, 74) with an opening (18, 78);
- a heat conductor (15, 66) at one side of the opening.

This makes it possible to concentrate any heat transfer from the heat conductor to the patient via the opening. Only the spot to be treated is heated. Adjacent skin areas are not harmed or affected.

Optionally, the thermally insulating layer may be provided with a self-adhesive layer on a side configured to be applied on a patient's skin. The opening through the insulating layer may for example at one side cross the self-adhesive layer, while the heat conductor is a heat conductive top layer covering the opposite side of the opening. If the adhesive carrier, the insulating layer and the heat conductive top layer are formed of flexible foil materials, then they jointly form a plaster, e.g., to be used as a disposable product.

Suitable materials for the self-adhesive carrier layer would for example include polyethylene foil, for instance with a medical grade acrylic adhesive. The insulating layer may for example comprise a foam, e.g., a polyethylene foam. Suitable materials for the heat conductive top layer would for example include aluminium foil.

The heat conductive top layer can be heated by a heating element, while it protects the heating element from viral infection or contamination by the wart or other skin disorder to be treated. The device may for example also comprise a docking station comprising a power source, a heating element configured to establish a releasable contact with the heat conductor, and a line connecting the heating element to the power source. The heating element may for example be sunk in a recess matching the outline of the heat conductor. This way, the heating element can accurately be positioned on top of the heat conductor.

If the heating element is sunk in a recess, it is advantageous if the self-adhesive layer of the plaster is wider than the insulating layer and the heat conductive layer of the plaster when it is in use. This way, the self-adhesive surface of the self-adhesive layer can stick to the wall surrounding the recess if the heating element, which helps to establish and maintain a reliable contact between the heat conductor and the wart.

In a specific embodiment the heating element of the docking station may for example comprise a flexible carrier strip with a printed track of electro-conductive heat generating material operatively connected or connectable to the power source, the carrier strip having one heat exchange end, which is shaped and sized to cooperate with the heat conductor. The heat exchange end may for example be an enlarged end section of the strip, e.g., a round or oval section where the printed track meanders to distribute heat over the surface of the enlarged end section. The carrier strip can be covered by a flexible cover strip, e.g., having a recess with a surrounding wall, the heat exchange end of the carrier strip being sunk within said recess with a depth corresponding to the height of the plaster.

To prevent overheating of the patient's skin, the heating element may be provided with at least one heat sensor, such as a thermistor, e.g., at the heat exchange end of the carrier strip. In a specific embodiment the heating element may have one or more of such sensors at opposite sides of the enlarged end section.

The docking station may for example comprise a strap or a similar binder to tie the docking station to a wrist or ankle of the patient. The strap may for example comprise Velcro fasteners or other releasable fasteners.

In an alternative embodiment, the heat conductor may comprise an activation metal in a heat pack and a protrusion projecting from the heat pack. The device of this embodiment may for example comprise an insulating pouch covering the heat pack, the pouch having an opening for passage of the protrusion of the heat conductor. This way, all heat generated by the heat pack is dissipated via the heat conductor. The device may further comprise an insulating part with a bore for receiving the protrusion of the heat conductor over a length that is shorter than the length of the bore, e.g., about 1 - 4 mm, e.g., about 2 - 3 mm shorter. The other end of the bore can then be positioned on a wart or other sport to be treated, in such a manner that the wart or spot contacts the heat conductor.

Optionally, the pouch and/or the insulating part are connected to a strap with the protrusion of the heat conductor extending through part of the bore of the insulating part. This makes it possible to tie the device to a wrist or ankle of a patient during treatment.

Heating pads may for example contain a supersaturated solution, e.g., an aqueous solution of sodium acetate. Crystallization is triggered by flexing a small metal part, e.g., of notched ferrous metal embedded in the supersaturated solution. Because the liquid is supersaturated, crystallization takes place rapidly and releases heat. Heat pads can be reused by placing it in boiling water for a while. This will also clean the insulating part placed on the wart and the heat conductor. Once the pad has cooled again to room temperature the device can be reused for a next treatment.

The invention also relates to a method of treating a skin disorder, such as a wart, by positioning a skin spot to be treated in a bore or opening in an insulating layer and by contacting the skin spot with a heat conductor which is heated by a heat source, such as a heat pack or an electric heating element.

The above-described aspects will hereafter be more explained with further details and benefits with reference to the drawings showing a number of embodiments by way of example.
Figure 1: shows a device according to the invention during treatment of a wart;
Figure 2: shows the plaster of the device of Figure 1;
Figure 3: shows the plaster in exploded view;
Figure 4: shows the docking station of the device of device of Figure 1;
Figure 5: shows the docking station in exploded view;
Figure 6: shows the carrier strip in bottom view;
Figure 7: shows the device in cross section in use;
Figure 8: an alternative embodiment in cross section;
Figure 9: shows the embodiment of Figure 6 in exploded view.

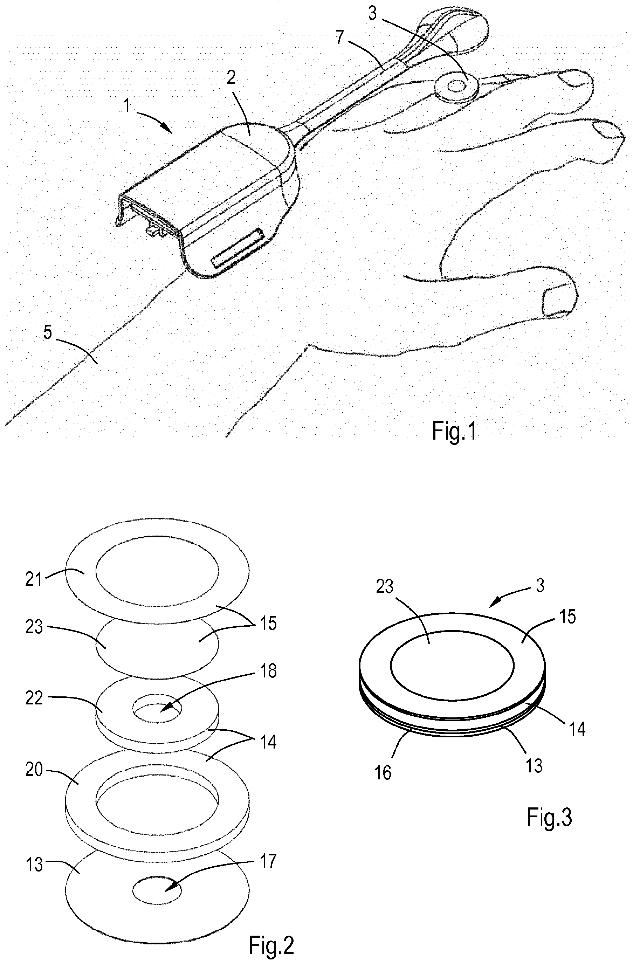

Figure 1 shows a device 1 for the treatment of skin disorders, in particular warts. The device 1 comprises a reusable docking station 2 and a disposable plaster 3. The plaster 3 is to be applied on a spot to be treated, e.g., over a wart. The docking station 2 can be tied to a patient, e.g., on his or her arm 5 or leg by means of a strap. A flexible line 7 connects the docking station 2 mechanically and electroconductively to a heating unit 9 which is placed over the plaster 11.

Figure 2 shows the plaster 3 before use, with the layers shown with relatively large thicknesses in order to show the complete build-up of the plaster. The plaster 3 comprises three layers: a self-adhesive base layer 13 to be applied onto the patient's skin, a foam layer 14 and a heat conductive top layer 15. The foam layer 14 is fixed to the base layer 13 by means of a releasable adhesive, while the top layer is adhered to the foam layer by means of a non-releasable adhesive layer.

As shown in the exploded view of Figure 3, the base layer 13 and the foam layer 14 comprise a central opening 17, 18 for receiving a wart to be treated, in such a manner that the wart's top end contacts the heat conductive top layer 15.

Both the top layer 15 and the foam layer 14 comprise an annular peripheral section 20, 21 and a circular core 22, 23 fitting within the annular peripheral section. The core 23 of the heat conductive top layer closes off the central opening 18 of the foam layer 14.

The self-adhesive base layer 13 is adhesive at both sides. The downside can be adhered to a patient, while its topside adheres to the foam layer 14 in a releasable manner.

Before use the self-adhesive downside of the base layer 13 is protected by a release paper 16(Figure 2). After removal of the release paper 16 the plaster 3 can be applied onto the patient's skin. After that the annular sections 20, 21 of the foam and top layers 14, 15 are peeled off jointly and thrown away. After removal of the annular sections 20, 21 the top surface of the base layer 13 is still adhesive.

In the shown embodiment the plaster 3 is circular. Alternatively, the plaster 3 may have any other suitable outline, e.g., oval or elliptical or the like.

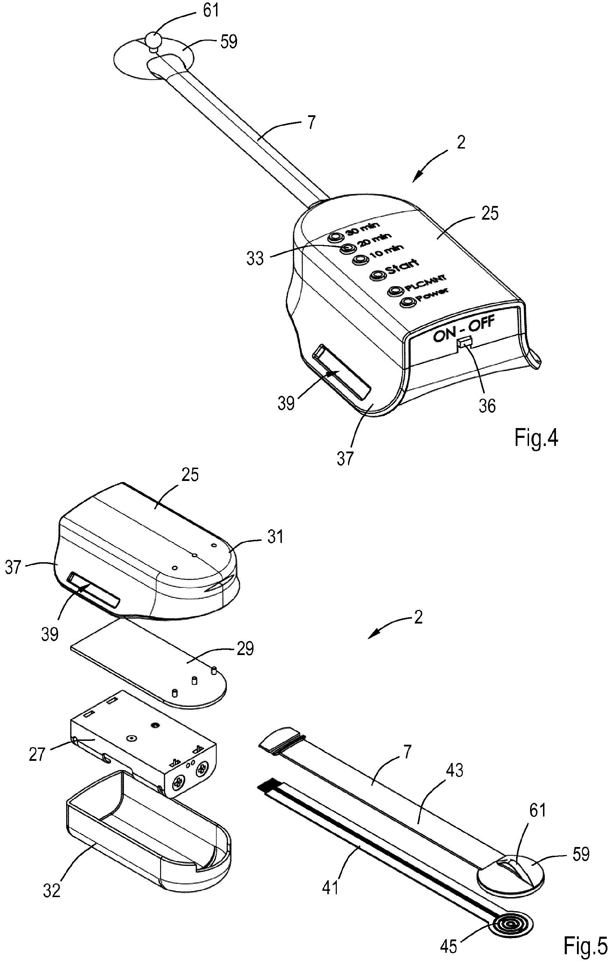

The docking station 2 (see Figure 5) comprises a housing 25, a rechargeable battery 27 fitting within the housing 25, and a circuit board 29, also fitting within the housing 25 and being configured to extract power at a desired voltage (e.g., about 3 V) from the battery 27 to pass it on to the flexible line 7. The housing 25 comprises an upper housing part 31 and a lower housing part 32 connected to each other.

In alternative embodiments, the docking station 2 may also comprises a timer 33, in this embodiment a set of three LED lights, the first LED starting to glow after 10 minutes of treatment, the second one after 20 minutes and, finally, the third one starting to glow after 30 minutes of treatment. Alternative, the docking station 2 may comprise a display at a top surface of the housing 25. The timer or display can be fed and controlled via circuitry on the circuit board 29. An on/off switch 36 allows a patient to turn on the heating element or to turn it off.

The side faces of the housing are provided by flat downward extensions 37 both provided with a slit 39 for attachment of a strap (not shown) for binding the device 1 to a patient, e.g., an arm or leg.

The flexible line 7 comprises a carrier strip 41 and a cover strip 43 for covering the carrier strip 41. The carrier strip 41 has one end connected to the circuit board 29 in the housing 25 and a free end with a circular section 45.

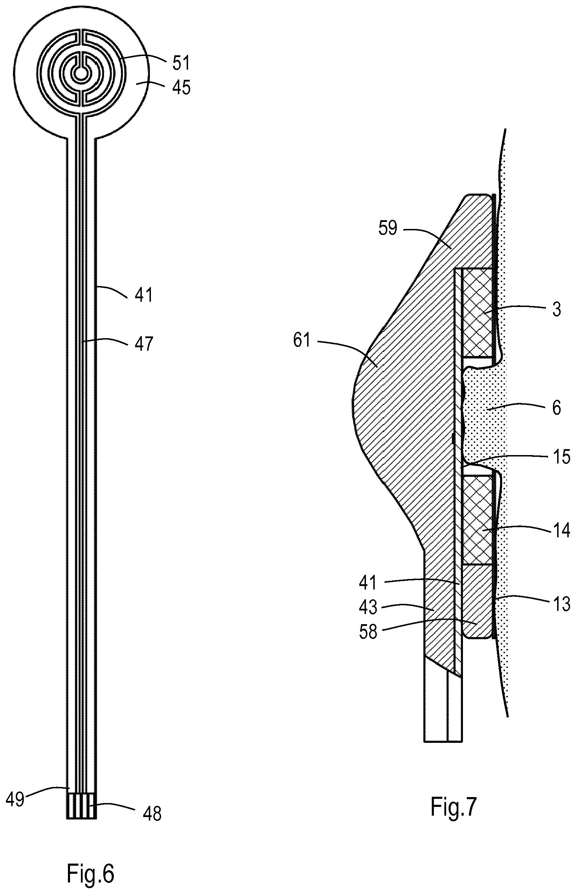

The carrier strip 41 has a bottom side (see Figure 7) provided with a printed electro-conductive track 47 with a feeding end 48 fed by the circuit board 29 and a return end 49 returning to the circuit board 29. Between the two ends 48, 49 the track 47 loops to the circular section 45 of the carrier strip 41 and back. From the feeding end 48 the track 47 extends in a straight line towards the circular section 45 where it meanders in a regular circular pattern to form a heating element 51, after which it return via a straight line to the return end 49. When a patient switches the device 1 on, an electric current follows the conductive track 47 and generates heat. When the patient switches it off the current stops and the carrier strip 41 cools down.

When the conductive top layer 15 of the plaster 3 touches the printed track 47 of the heating element 51 the impedance of the track 47 is changed, which is detected by the connected electronics. This way, it possible to check whether or not the heating element is correctly positioned on top of the plaster 3.

The resistance of the track 47 will also change with the temperature of the track 47. This makes it possible to monitor possible overheating and/or to adjust the electric power in order to maintain the temperature at a desired level.

The cover strip 43 is flexible and has a flat top side with a circular section 59 at one end of the cover strip 43. The circular section 59 is provided with a grip 61 to allow easier handling and positioning. At its lower side the cover strip 43 has a recess 56 for receiving the carrier strip 41. At the position of the circular section 59 the recess 56 is somewhat deeper surrounded by a circular wall 58 so it can receive simultaneously the circular section of the carrier strip 41 with the heating element 51 and the plaster 3 (see Figure 7).

After the plaster 3 is applied over a wart 6 to be treated and the annular sections 20, 21 of the top and foam layers 14, 15 are peeled off, the circular section 59 of the flexible line 7 is positioned over the plaster 3, such that the plaster 3 is received in the recess 56 of the circular section 59, as shown in Figure 7. The annular wall 58 around the recess of the circular section 59 contacts the annular self-adhesive top surface of the base layer 13 of the plaster 3 which helps to keep the circular section 59 in place during use. In that position the heating element 51 of the flexible line 7 contacts the heat conductive top layer 15 of the plaster 3 which in turn contacts the wart 6 received in the central apertures 17, 18 of the foam and base layers 13, 14 of the plaster 3. The heat conductive top layer 15 is able to transfer heat from the heating element 51 and protects the heating element 51 from viral infection or contamination by the wart. The patient can switch on the device 1 to heat the heating element 51, which in turn heats the wart 6 via the conductive top layer 15 of the plaster 3. A heating temperature of about 44 - 50°C can for example be used to treat the wart successfully without damaging surrounding skin tissue.

After a given time, e.g., after about 30 minutes, or after detection of a limit temperature, the heating element 51 is switched off. The circular section 59 of the flexible line 7 can be removed from the plaster 3 and be cleaned. The plaster 3 can be removed and disposed.

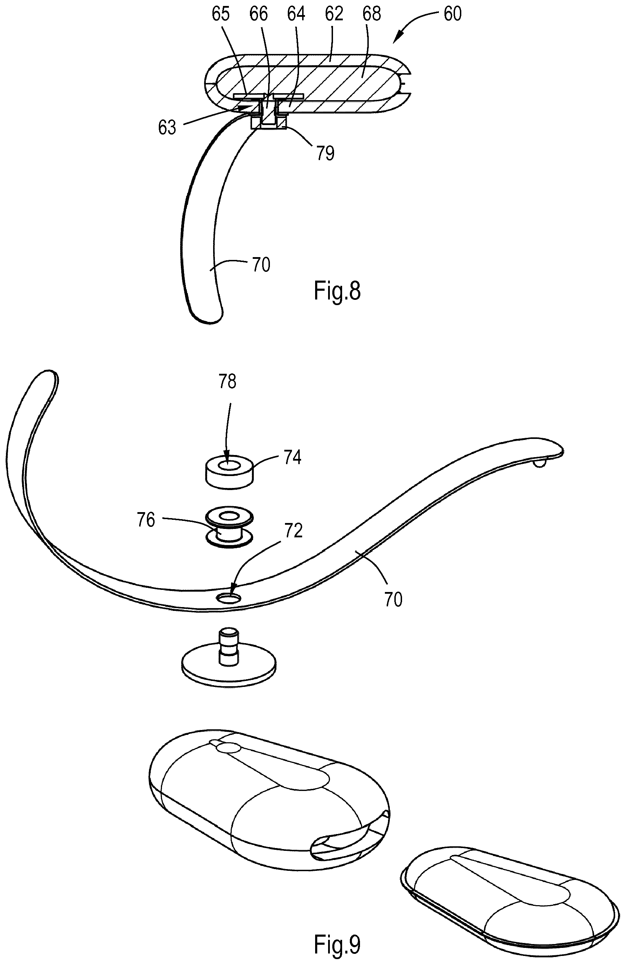

Figures 8 and 9 show an alternative embodiment. This embodiment comprises a device 60 for the treatment of skin disorders. The device 60 comprises a pouch 62 of a thermally insulating material, such as neoprene, with an opening 63 at one side of the pouch 62. The device 60 further comprises a heat conductor 64 having a disk section 65 inside the pouch 62 and a protrusion 66 extending from the disk section 65 through the opening 64 in the pouch 62. A matching heat pack 68 filled with a suitable heat pack gel can be fit into the pouch 62 and tightly engage heat conductor 64.

The device of Figures 8 and 9 also comprises a strap 70 which can be tied around an arm or leg of a patient. The strap 70 has an opening 72. A cylindrical foam part 74 is aligned with the opening 72 in the strap 70 and adhered to a plastic clicker 76, which is clicked into the opening in the strap 70. The cylindrical foam part 74 has a central bore 78 sized to receive a wart to be treated.

When a patient wishes to treat a wart he places a fresh heat pack 68 into the pouch 62, puts the cylindrical foam part 74 over the wart and ties the strap 70 around the infected body part. Then he inserts the projection 66 of the heat conductor 64 into the opening 72 of the strap 70 and the cylindrical foam part 74 until it contacts the wart. Pressing the disk 65 of the heat conductor 64 triggers crystallization of the contents of the heat pack 68. This generates heat. Due to the isolating pouch 62 the heat can only be dissipated via the protrusion 66 of the heat conductor 64. As a result all heat is focused on the wart. After a while the pouch 62 can be removed and the strap 70 can be untied and cleaned.

## Claims

1. A device (1, 60) for the treatment of skin disorders the device comprising:
- a layer of a thermally insulating material (14, 74) with an opening (18, 78);
- a heat conductor (15, 66) at one side of the opening.

2. The device of claim 1 wherein the thermally insulating layer (14) is provided with a self-adhesive layer (13) on a side configured to be applied on a patient's skin.

3. The device of claim 2, wherein the opening (18) crosses the self-adhesive carrier (13) and the heat conductor is a heat conductive top layer (15) covering one side of the opening.

4. The device of claim 3, the layers being formed of flexible foil materials jointly forming a plaster.

5. The device of any preceding claim, further comprising a docking station comprising a power source (27), an heating element (51) configured to establish a releasable contact with the heat conductor (15), and a line (7) connecting the heating element to the power source.

6. The device of claim 5, wherein the heating element (51) is sunk in a recess matching the outline of the heat conductor (15).

7. The device of claim 5 or 6, wherein the heating element comprises a flexible carrier strip with a printed track of electro-conductive heat generating material operatively connected or connectable to the power source, the carrier strip having one heat exchange end, which is shaped and sized to cooperate with the heat conductor (15).

8. The device of claim 5, 6 or 7, wherein the heating element comprises at least one heat sensor, such as a thermistor, e.g., at the heat exchange end of the carrier strip.

9. The device of claim 5, 6, 7 or 8, wherein the docking station comprises a strap.

10. The device (60) of claim 1, wherein the heat conductor comprises an activation metal (65) in a heat pack (68) and a protrusion (66) projecting from the heat pack.

11. The device of claim 10, comprising an insulating pouch (62) covering the heat pack, the pouch having an opening (63) for passage of the protrusion of the heat conductor.

12. The device of claim 10 or 11, comprising an insulating part (74) with a bore for receiving the protrusion (66) of the heat conductor over a length that is shorter than the length of the bore.

13. The device of claim 11 or 12, wherein the pouch and/or the insulating part are connected to a strap (70) with the protrusion (66) of the heat conductor extending through part of the bore of the insulating part (74).
